# EUROPEAN PATENT SPECIFICATION

(11) **EP 3 613 851 B1**
(45) Date of publication and mention of the grant of the patent: **04.11.2026**
(21) Application number: 17889883.9
(22) Date of filing: 09.01.2017
(51) Int. Cl.: C12N 9/12

(54) **RECOMBINANT DNA POLYMERASE**
REKOMBINANTE DNA-POLYMERASE
ADN POLYMÉRASE RECOMBINANTE

(43) Date of publication of application: 26.02.2020
(73) Proprietor: MGI Tech Co., Ltd., Yantian District Shenzhen Guangdong 518083 (CN)
(72) Inventor: WANG, Lin, Shenzhen Guangdong 518083 (CN); LIU, Fen, Shenzhen Guangdong 518083 (CN); DONG, Yuliang, Shenzhen Guangdong 518083 (CN); ZHANG, Wenwei, Shenzhen Guangdong 518083 (CN); XU, Chongjun, Shenzhen Guangdong 518083 (CN); DRMANAC, Snezana, Shenzhen Guangdong 518083 (CN)
(74) Representative: karo IP
(86) International application number: PCT/CN2017/070609
(87) International publication number: WO 2018/126470

(56) References cited:
- WO-A1-2015/200693
- WO-A1-2016/054096
- WO-A2-2009/131919
- CN-A- 104 854 237
- ANONYMOUS: "GSP:AXS13274; Thermococcus sp. DNA polymerase mutant, L408P/Y409A/S411T/A485L.", GENESEQ PROTEINS, 10 December 2009 (2009-12-10), XP055696364, Retrieved from the Internet <URL:http://ibis.internal.epo.org/exam/dbfetch.jsp?id=GSP:AXS13274> [retrieved on 20200518]
- ANONYMOUS: "EPOP:LQ304881; Sequence 12 from Patent WO2016054096", 4 October 2016 (2016-10-04), XP055696373, Retrieved from the Internet <URL:http://ibis.internal.epo.org/exam/dbfetch.jsp?id=EPOP:LQ304881> [retrieved on 20200518]
- HASHIMOTO, H: "Crystal structure of DNA polymerase from hyperthermophilic archaeon Pyrococcus kodakaraensis KODI", JOURNAL OF MOLECULAR BIOLOGY, vol. 306, no. 3, 23 February 2001 (2001-02-23), pages 469 - 477, XP004469251, ISSN: 0022-2836

## Description

### FIELD

The present disclosure relates to the field of biotechnology, particularly to a recombinant DNA polymerase.

### BACKGROUND

DNA polymerase catalyzes the rapid and accurate replication of DNA in chromosomes of a living organism, which plays an important role in maintaining the stability of genetic materials in the living organism. The *Escherichia coli* (E. coli) based polymerases, mainly including polymerases I, II and III, are respectively divided into three families A, B and C due to their difference in amino acid sequences. Despite their similar nucleotide binding sites in structure, the polymerases exhibit different recognition mechanisms for a nucleotide and its analogues because they have significant different motifs. The polymerase which is tightly associated with the activities in a living organism, is thus in a core position for molecular biology technologies, especially in the aspects of molecular cloning, PCR reaction, site-directed mutation, and DNA sequencing. For the molecular biology technologies, DNA polymerase is critical for the recognition of a nucleotide and its analogues or the binding of the nucleotide to a template.

Many practical applications rely on the DNA polymerase which is capable of accurately recognizing the nucleotide or its analogs added. For example, for the sequencing-by-synthesis (SBS) with high throughput and low price compared to other current sequencing methods, the nucleotide which is labeled with fluorescein benefits the identification of the bases of a DNA template, thereby obtaining the base sequences of the DNA template. During the SBS method, the addition of DNA polymerase recognizing an artificially modified nucleotide or its nucleotide analogs (such as a reversible terminator with a fluorescent label) is critical. In current DNA polymerization reaction, the DNA polymerase in the prior art which cannot polymerize the artificially modified nucleotide or its nucleotide analogs effectively has limited the replication of DNA. Thus, it is important to increase the polymerization efficiency of DNA polymerase to polymerize the artificially modified nucleotide or its nucleotide analogs, especially for the sequencing method.

WO 2009/131919 A2 discloses Compositions and methods are provided that relate to a recombinant protein with DNA polymerase activity in which one or more amino acids are mutated compared with the corresponding wild type protein. The recombinant protein is capable of incorporating one or more modified nucleotides into a nucleic acid substrate with a specific activity greater than 200.

WO 2016/054096 A1 relates to polymerase enzymes for improved incorporation of nucleotide analogues, in particular nucleotides which are modified at the 3' sugar hydroxyl, as well as methods and kits using the same.

### SUMMARY

The protein defined in the claims and provided in the present disclosure is
a protein that has DNA polymerase activity and which is characterised by having improved catalytic and physicochemical properties in comparison to a wild-type KOD DNA polymerase of SEQ ID NO: 1, and
wherein the protein has an amino acid sequence which includes a mutation at position 408 from leucine to isoleucine, a mutation at position 409 from tyrosine to alanine, a mutation at position 485 from alanine to glutamic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine as compared to an amino acid sequence of the wild-type KOD DNA polymerase of SEQ ID NO: 1; or has an amino acid sequence which includes a mutation at position 408 from leucine to isoleucine, a mutation at position 409 from tyrosine to alanine, a mutation at position 485 from alanine to glutamic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine as compared to an amino acid sequence of the wild-type KOD DNA polymerase of SEQ ID NO: 1.

According to the protein describe above, the wild-type KOD DNA polymerase is of an amino acid sequence depicted as SEQ ID NO: 1.

According to the protein describe above, the protein has a lower adsorption capacity for a biochip compared to the wild-type KOD DNA polymerase.

According to the protein describe above, the lower adsorption capacity for the biochip is reflected in that the protein is poor at adsorption on the surface of the biochip and/or is easy to be eluted from the surface of the biochip.

A DNA molecule encoding the protein described above is within the scope of the present disclosure; or
an expression cassette, a recombinant expression vector, a recombinant bacteria or a transgenic cell line containing the DNA molecule described above are also within the scope of the present disclosure.

Use of the protein, the DNA molecule, the expression cassette, the recombinant expression vector, the recombinant bacteria or the transgenic cell line described above for preparing a DNA polymerase is also within the scope of the present disclosure.

According to the use described above, the DNA polymerase has a lower adsorption capacity for a biochip compared to a wild-type KOD DNA polymerase; and
the lower adsorption capacity for the biochip is reflected in that the DNA polymerase is poorer at adsorption on the surface of the biochip and/or is easier to be eluted from the surface of the biochip compared to the wild-type KOD DNA polymerase.

Use of the protein, the DNA molecule, the expression cassette, the recombinant expression vector, the recombinant bacteria or the transgenic cell line described above in sequencing is within the scope of the present disclosure; or
use of the protein, the DNA molecule, the expression cassette, the recombinant expression vector, the recombinant bacteria or the transgenic cell line described above in preparing a product for sequencing is also within the scope of the present disclosure.

According to the use described above, the product is a kit.

According to the protein described above, a_substrate of the protein served as a DNA polymerase is a nucleotide or its analogs.

According to the protein described above, a template for the protein served as a DNA polymerase is a single-stranded DNA.

According to the use described above, a substrate of the protein served as a DNA polymerase is a nucleotide or its analogs.

According to the use described above, a template for the protein served as a DNA polymerase is a single-stranded DNA.

According to the use described above, the protein served as a DNA polymerase is characterized by a high polymerization activity and/or a low adsorption for a biochip when a single-stranded DNA is used as a template.

According to the use described above, the high polymerization activity is reflected in that the protein served as a DNA polymerase has a higher polymerization activity than the wide-type KOD DNA polymerase;

According to the use described above, a polymerization reaction is conducted on a biochip.

The biochip described above can be any biochip commonly used in the art.

According to the use described above, the product is a kit.

The protein described above also refers to a variant protein of KOD DNA polymerase, specifically including:
a KOD DNA polymerase variant, including Ue1A, Ue1B, Ue1C, Ue1D, Ue1E, Ue2A, Ue2B, Ue2C, Ue2D, Ue2E, Ue3A, Ue3B, Ue3C, Ue3D, Ue3E, Ue4A, Ue4B, Ue4C, Ue1A5, Ue1A6, Ue1B5, Ue1B6, Ue1C5, Ue1C6, Ue1D5, Ue1D6, Ue1E5, Ue1E6, Ue2A5, Ue2A6, Ue2B5, Ue2B6, Ue2C5, Ue2C6, Ue2D5, Ue2D6, Ue2E5, Ue2E6, Ue3A5, Ue3A6, Ue3B5, Ue3B6, Ue3C5, Ue3C6, Ue3D5, Ue3D6, Ue3E5, Ue3E6, Ue4A5, Ue4A6, Ue4B5, Ue4B6, Ue4C5 and Ue4C6, and
a C-terminal shortened KOD DNA polymerase variant, being the KOD DNA polymerase variant described above of which 29 amino acids at the C-terminal region is deleted and the remaining amino acids are kept unchanged.

The present disclosure is accomplished based on the following discoveries: a series of variants of KOD DNA polymerase with improved catalytic and physicochemical properties are generated, by mutating amino acids in some sites of the wide-type KOD DNA polymerase through semi-rational design, enzyme variant library construction and high-throughput screening, based on dynamic simulation of three-dimensional structures of naturally occurring DNA polymerases and their artificially modified variants and statistical inference of sequence information of the DNA polymerases. Such the variants obtained are poor at adsorbing on the surface of a chip and are easily eluted from the surface of the chip, thereby being suitable for DNA sequencing in a sequencing chip.

### BRIEF DESCRIPTION OF THE DRAWINGS

Figure 1 is a schematic graph showing the electrophoresis result of purification detection of purified wild-type KOD DNA polymerase.
Figure 2 is a schematic graph showing the Michaelis kinetics curves of the wild-type KOD DNA polymerase and its variants Ue1E, Ue1E5 and Ue1E6 in a substrate of a single-stranded DNA.
Figure 3 is a schematic graph showing bright spots of the wild-type KOD DNA polymerase and its variant Ue1E5 present on a chip at an activity assay.
Figure 4 is a schematic graph showing bright spots of the wild-type KOD DNA polymerase variant Ue1E5 and Therminator DNA polymerase as a control present on a chip at an elution assay.

### DETAILED DESCRIPTION

The experimental methods used in the following examples are conventional methods unless otherwise specified.

The materials, reagents and the like used in the following examples are commercially available unless otherwise specified.

### Examples

### Example 1. Preparation of wild-type KOD DNA polymerase and its variants

In this example, an expressing vector of the wild-type KOD DNA polymerase and its variants are constructed by using Electra^{™} Cloning Reagents Kit (DNA 2.0), with a His-tag for affinity purification on a Ni column.

### 1.1 Preparation of fusion protein of wild-type KOD DNA polymerase

The wild-type KOD DNA polymerase is of an amino acid sequence as depicted in SEQ ID NO: 1, which is encoded by its encoding gene *phaP* having a nucleotide sequence as depicted in SEQ ID NO: 2.

### 1.1.1 Construction of recombinant expression vector pD441-WT

The recombinant expression vector pD441-WT was obtained by recombining an encoding gene of a fusion protein of the wild-type KOD DNA polymerase with a His-tag into the vector pD441-pelB (DNA2.0, pD441-pelB) according to the instruction of Electra^{™} Cloning Reagents Kit (DNA2.0, EKT-02), where the expression of such the encoding gene was directed by a signal peptide of the vector pD441-pelB.

The fusion protein of the wild-type KOD DNA polymerase with the His-tag is of an amino acid sequence which connects with 6 His-tags at its C-terminus compared to the amino acid sequence of the wild-type KOD DNA polymerase as depicted in SEQ ID NO: 1.

The encoding gene of the fusion protein of the wild-type KOD DNA polymerase with the His-tag is of a nucleotide sequence which connects with codons of 6 His-tags at its 3' end compared to the nucleotide sequence of the wild-type KOD DNA polymerase as depicted in SEQ ID NO:2.

### 1.1.2 Construction of recombinant bacteria

After transfected with the recombinant expression vector pD441-WT, the *E. coli* BL21 competent cells purchased from TransGen Biotech were plated into a petri dish containing 50µg/mL kanamycin to screen positive colonies. 3 to 5 positive colonies picked out were subjected to the colony PCR amplification under a pair of primers (i.e. SQF depicted in SEQ ID NO: 3, and SQR depicted in SEQ ID NO: 4), thus obtaining the positive colony loaded with the encoding gene in a length of 2800 bp identical to the theoretical value expected and named as the positive colony BL21/pD441-WT.

### 1.1.3 Expression and purification of fusion protein of wild-type KOD DNA polymerase

A single positive colony BL21/pD441-WT picked out was inoculated in 50 mL LB liquid culture medium containing 50µg/mL kanamycin for culture at 37°C overnight, with 220 rpm/min of shaking. In the next day, the bacteria solution in a 1:100 dilution was transferred to 1000 mL LB liquid culture medium with 50µg/mL kanamycin and cultured at the same condition until the value of OD600 reaches 0.5 to 0.8. After that, an inducer Isopropyl β-D-Thiogalactoside (IPTG) in a final concentration of 0.5mM was added to induce the expression of recombinant expression vector, and the bacteria solution was still cultured at 25°C overnight, followed that the bacteria solution BL21/pD441-WT was harvested, while the bacteria solution without the inducer IPTG was used as a blank control, and the corresponding uninduced bacteria solution BL21/pD441-WT was also collected.

The induced bacterial solution BL21/pD441-WT was centrifuged at 8000 rpm/min for 10 min, followed by removing the supernatant, thus obtaining the precipitated pellets. The bacterial pellets were subsequently resuspended in a first buffer (including 50 mMKPO₄, 500 mM NaCl, 10 mM imidazole and 5% Glycerol, PH 7.0), to which Phenylmethanesulfonyl fluoride (PMSF) in a final concentration of 0.5mM, Triton X-100 in a final concentration of 0.5% and Lysozyme in a final concentration of 0.25% were added, followed by incubating at room temperature for 30 minutes. After that, the bacterial solution was subjected to centrifugation at 12000rpm/min and 4°C for another 30min, an ice bath, ultrasonication to break bacterial cells, and centrifugation at 12000rpm/min for further 30min, with the supernatant collected warming in a water bath of 75°C for 20 minutes, during which mixing was taken after a definite time period to ensure uniform heating. After centrifugation at 12000rpm/min and 4°C for 30min, the supernatant was filtered through a 0.22 um filter to obtain a crude fusion protein.

The crude fusion protein was loaded into a Ni column for affinity chromatography (HisTrap FF, 5 mL, 17-5255-01, GE healthcare) at a suitable flow rate, after which 5 column volumes (CVs) of the first buffer was loaded for equilibration, and 5 CVs of a second buffer in 3% (including 50 mMKPO₄, 1M NaCl and 5% Glycerol, PH 7.0) loaded for a first elution, 5 CVs of the second buffer in 50% loaded for a second elution, with collection of the eluate of the Ni column corresponding to a peak value of 100 mAU or above.

The eluate corresponding to the peak value of 100 mAU or above was loaded into an ion exchange column (HiTrap Q HP, 5mL, 17-1154-01, GE healthcare) at a suitable flow rate, after which 5 CVs of the second buffer was loaded for equilibration, and the second buffer from 0% to 60% loaded for a linear elution, with collection of the eluate of ion exchange chromatography corresponding to a peak value of 100 mAU or above.

The eluate of ion exchange chromatography corresponding to the peak value of 100 mAU or above was loaded into a gel chromatography column (HiPrep Sephacryl S-100 HR, 26mm, 17-1194-01, GE healthcare), where the gel chromatography column was washed with 3 CVs of 20% ethanol and 3CVs of water, and equilibrated with 3CVs of a third buffer in 100% (including 20 mM Tris, 200 mM KCl, 0.2 mM EDTA and10% Glycerol, PH7.4) before loaded with the eluate. After that, 1.5 CVs of the third buffer was loaded for elution, with the eluate (that is a purified fusion protein) collected.

The purified fusion protein of the wild-type KOD DNA polymerase was detected to be a yield of 40mg/L.

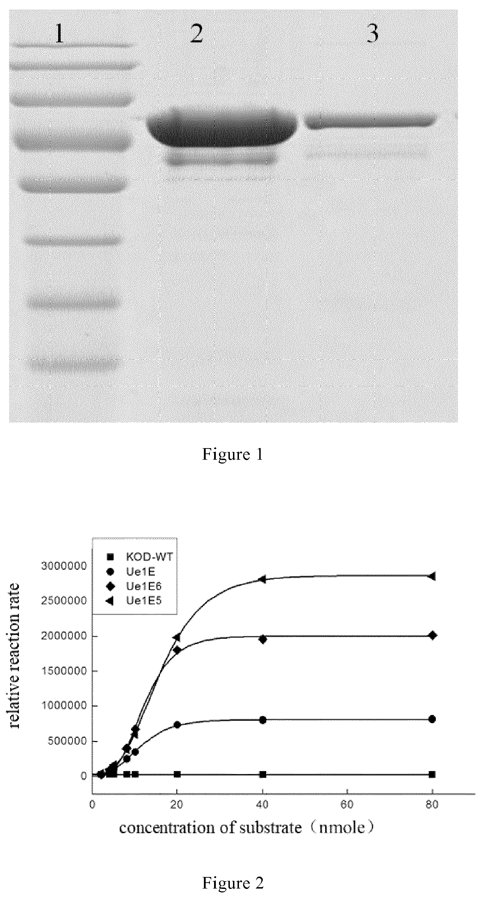

The purified fusion protein of the wild-type KOD DNA polymerase was subjected to SDS-PAGE gel electrophoresis (where 5% of concentrated gel and 12% of separation gel were used). The results are shown in Figure 1, in which Lane 1 represents a marker (PageRuler Prestained Protein Ladder, 26616, Thermo Scientific), Lane 2 represents a mixture of the purified fusion protein of the wild-type KOD DNA polymerase (1 mg/mL, 10µL) and 2×loading buffer (10µL), and Lane 3 represents a mixture of the purified fusion protein of the wild-type KOD DNA polymerase (0.05 mg/mL, 10µL) and 2×loading buffer (10µL), and it can be seen that the protein occurring in Lane 2 and Lane 3 is of a molecular weight about 91.5 KDa respectively, which is consistent with that reported in a literature.

The gel containing the purified fusion protein after gel electrophoresis was taken and detected by the Quantity one software for purity, and the result shows that the purity of the purified fusion protein is 95% or above.

This example also shows that no purified fusion protein in a molecular weight of about 91.5 KDa is present in the uninduced bacterial solution BL21/pD441-WT.

In this example, an empty vector pD441-pelB without the recombinant expression vector pD441-WT was also transfected into *E. coli* BL21, with the colony BL21/pD441-pelB was obtained, which was then subjected to protein expression and purification as the method described in 1.1.3, and no purified fusion protein in a molecular weight of about 91.5 KDa was detected similarly.

### 1.2 Preparation of fusion protein of KOD DNA polymerase variant

a KOD DNA polymerase variant refers to a protein having at least three of amino acid substitutions at positions 53, 59, 199, 243, 408, 409, 485, 526, 558, 613, 641, 671, 673, 674, 692 and 709 compared to the amino acid sequence of a wild-type KOD DNA polymerase depicted in SEQ ID NO: 1. The specific variants showed below in this example have the following mutations.

Ue1A, has an amino acid sequence which includes a mutation at position 408 from leucine to isoleucine, a mutation at position 409 from tyrosine to alanine, and a mutation at position 485 from alanine to histidine compared to the amino acid sequence depicted in SEQ ID NO: 1;

Ue1B, has an amino acid sequence which includes a mutation at position 408 from leucine to isoleucine, a mutation at position 409 from tyrosine to alanine, and a mutation at position 485 from alanine to asparagine compared to the amino acid sequence depicted in SEQ ID NO: 1;

Ue1C, has an amino acid sequence which includes a mutation at position 408 from leucine to isoleucine, a mutation at position 409 from tyrosine to alanine, and a mutation at position 485 from alanine to lysine compared to the amino acid sequence depicted in SEQ ID NO: 1;

Ue1D, has an amino acid sequence which includes a mutation at position 408 from leucine to isoleucine, a mutation at position 409 from tyrosine to alanine, and a mutation at position 485 from alanine to aspartic acid compared to the amino acid sequence depicted in SEQ ID NO: 1;

Ue1E, has an amino acid sequence which includes a mutation at position 408 from leucine to isoleucine, a mutation at position 409 from tyrosine to alanine, and a mutation at position 485 from alanine to glutamic acid compared to the amino acid sequence depicted in SEQ ID NO: 1;

Ue2A, has an amino acid sequence which includes a mutation at position 408 from leucine to alanine, a mutation at position 409 from tyrosine to alanine, and a mutation at position 485 from alanine to histidine compared to the amino acid sequence depicted in SEQ ID NO: 1;

Ue2B, has an amino acid sequence which includes a mutation at position 408 from leucine to alanine, a mutation at position 409 from tyrosine to alanine, and a mutation at position 485 from alanine to asparagine compared to the amino acid sequence depicted in SEQ ID NO: 1;

Ue2C, has an amino acid sequence which includes a mutation at position 408 from leucine to alanine, a mutation at position 409 from tyrosine to alanine, and a mutation at position 485 from alanine to lysine compared to the amino acid sequence depicted in SEQ ID NO: 1;

Ue2D, has an amino acid sequence which includes a mutation at position 408 from leucine to alanine, a mutation at position 409 from tyrosine to alanine, and a mutation at position 485 from alanine to aspartic acid compared to the amino acid sequence depicted in SEQ ID NO: 1;

Ue2E, has an amino acid sequence which includes a mutation at position 408 from leucine to alanine, a mutation at position 409 from tyrosine to alanine, and a mutation at position 485 from alanine to glutamic acid compared to the amino acid sequence depicted in SEQ ID NO: 1;

Ue3A, has an amino acid sequence which includes a mutation at position 408 from leucine to valine, a mutation at position 409 from tyrosine to alanine, and a mutation at position 485 from alanine to histidine compared to the amino acid sequence depicted in SEQ ID NO: 1;

Ue3B, has an amino acid sequence which includes a mutation at position 408 from leucine to valine, a mutation at position 409 from tyrosine to alanine, and a mutation at position 485 from alanine to asparagine compared to the amino acid sequence depicted in SEQ ID NO: 1;

Ue3C, has an amino acid sequence which includes a mutation at position 408 from leucine to valine, a mutation at position 409 from tyrosine to alanine, and a mutation at position 485 from alanine to lysine compared to the amino acid sequence depicted in SEQ ID NO: 1;

Ue3D, has an amino acid sequence which includes a mutation at position 408 from leucine to valine, a mutation at position 409 from tyrosine to alanine, and a mutation at position 485 from alanine to aspartic acid compared to the amino acid sequence depicted in SEQ ID NO: 1;

Ue3E, has an amino acid sequence which includes a mutation at position 408 from leucine to valine, a mutation at position 409 from tyrosine to alanine, and a mutation at position 485 from alanine to glutamic acid compared to the amino acid sequence depicted in SEQ ID NO: 1;

Ue4A, has an amino acid sequence which includes a mutation at position 408 from leucine to serine, a mutation at position 409 from tyrosine to alanine, and a mutation at position 485 from alanine to histidine compared to the amino acid sequence depicted in SEQ ID NO: 1;

Ue4B, has an amino acid sequence which includes a mutation at position 408 from leucine to serine, a mutation at position 409 from tyrosine to alanine, and a mutation at position 485 from alanine to asparagine compared to the amino acid sequence depicted in SEQ ID NO: 1;

Ue4C, has an amino acid sequence which includes a mutation at position 408 from leucine to serine, a mutation at position 409 from tyrosine to alanine, and a mutation at position 485 from alanine to lysine compared to the amino acid sequence depicted in SEQ ID NO: 1;

Ue1A5, has an amino acid sequence which includes a mutation at position 408 from leucine to isoleucine, a mutation at position 409 from tyrosine to alanine, a mutation at position 485 from alanine to histidine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine and a mutation at position 709 from arginine to methionine compared to the amino acid sequence depicted in SEQ ID NO: 1;

Ue1A6, has an amino acid sequence which includes a mutation at position 408 from leucine to isoleucine, a mutation at position 409 from tyrosine to alanine, a mutation at position 485 from alanine to histidine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue1B5, has an amino acid sequence which includes a mutation at position 408 from leucine to isoleucine, a mutation at position 409 from tyrosine to alanine, a mutation at position 485 from alanine to asparagine, a mutation at position 53 from lysine to methionine, a mutation at position 199 from lysine to methionine, a mutation at position243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue1B6, has an amino acid sequence which includes a mutation at position 408 from leucine to isoleucine, a mutation at position 409 from tyrosine to alanine, a mutation at position 485 from alanine to asparagine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue1C5, has an amino acid sequence which includes a mutation at position 408 from leucine to isoleucine, a mutation at position 409 from tyrosine to alanine, a mutation at position 485 from alanine to lysine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine, compared to the amino acid sequence depicted in SEQ ID NO: 1;

Ue1C6, has an amino acid sequence which includes a mutation at position 408 from leucine to isoleucine, a mutation at position 409 from tyrosine to alanine, a mutation at position 485 from alanine to lysine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue1D5, has an amino acid sequence which includes a mutation at position 408 from leucine to isoleucine, a mutation at position 409 from tyrosine to alanine, a mutation at position 485 from alanine to aspartic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine, compared to the amino acid sequence depicted in SEQ ID NO: 1;

Ue1D6, has an amino acid sequence which includes a mutation at position 408 from leucine to isoleucine, a mutation at position 409 from tyrosine to alanine, a mutation at position 485 from alanine to aspartic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue1E5, has an amino acid sequence which includes a mutation at position 408 from leucine to isoleucine, a mutation at position 409 from tyrosine to alanine, a mutation at position 485 from alanine to glutamic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue1E6, has an amino acid sequence which includes a mutation at position 408 from leucine to isoleucine, a mutation at position 409 from tyrosine to alanine, a mutation at position 485 from alanine to glutamic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue2A5, has an amino acid sequence which includes a mutation at position 408 from leucine to alanine, a mutation at position 409 from tyrosine to alanine, a mutation at position 485 from alanine to histidine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue2A6, has an amino acid sequence which includes a mutation at position 408 from leucine to alanine, a mutation at position 409 from tyrosine to alanine, a mutation at position 485 from alanine to histidine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue2B5, has an amino acid sequence which includes a mutation at position 408 from leucine to alanine, a mutation at position 409 from tyrosine to alanine, a mutation at position 485 from alanine to asparagine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue2B6, has an amino acid sequence which includes a mutation at position 408 from leucine to alanine, a mutation at position 409 from tyrosine to alanine, a mutation at position 485 from alanine to asparagine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue2C5, has an amino acid sequence which includes a mutation at position 408 from leucine to alanine, a mutation at position 409 from tyrosine to alanine, a mutation at position 485 from alanine to lysine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue2C6, has an amino acid sequence which includes a mutation at position 408 from leucine to alanine, a mutation at position 409 from tyrosine to alanine, a mutation at position 485 from alanine to lysine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue2D5, has an amino acid sequence which includes a mutation at position 408 from leucine to alanine, a mutation at position 409 from tyrosine to alanine, a mutation at position 485 from alanine to aspartic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue2D6, has an amino acid sequence which includes a mutation at position 408 from leucine to alanine, a mutation at position 409 from tyrosine to alanine, a mutation at position 485 from alanine to aspartic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue2E5, has an amino acid sequence which includes a mutation at position 408 from leucine to alanine, a mutation at position 409 from tyrosine to alanine, a mutation at position 485 from alanine to glutamic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue2E6, has an amino acid sequence which includes a mutation at position 408 from leucine to alanine, a mutation at position 409 from tyrosine to alanine, a mutation at position 485 from alanine to glutamic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue3A5, has an amino acid sequence which includes a mutation at position 408 from leucine to valine, a mutation at position 409 from tyrosine to alanine, a mutation at position 485 from alanine to histidine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue3A6, has an amino acid sequence which includes a mutation at position 408 from leucine to valine, a mutation at position 409 from tyrosine to alanine, a mutation at position 485 from alanine to histidine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue3B5, has an amino acid sequence which includes a mutation at position 408 from leucine to valine, a mutation at position 409 from tyrosine to alanine, a mutation at position 485 from alanine to asparagine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue3B6, has an amino acid sequence which includes a mutation at position 408 from leucine to valine, a mutation at position 409 from tyrosine to alanine, a mutation at position 485 from alanine to asparagine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue3C5, has an amino acid sequence which includes a mutation at position 408 from leucine to valine, a mutation at position 409 from tyrosine to alanine, a mutation at position 485 from alanine to lysine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue3C6, has an amino acid sequence which includes a mutation at position 408 from leucine to valine, a mutation at position 409 from tyrosine to alanine, a mutation at position 485 from alanine to lysine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue3D5, has an amino acid sequence which includes a mutation at position 408 from leucine to valine, a mutation at position 409 from tyrosine to alanine, a mutation at position 485 from alanine to aspartic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue3D6, has an amino acid sequence which includes a mutation at position 408 from leucine to valine, a mutation at position 409 from tyrosine to alanine, a mutation at position 485 from alanine to aspartic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue3E5, has an amino acid sequence which includes a mutation at position 408 from leucine to valine, a mutation at position 409 from tyrosine to alanine, a mutation at position 485 from alanine to glutamic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue3E6, has an amino acid sequence which includes a mutation at position 408 from leucine to valine, a mutation at position 409 from tyrosine to alanine, a mutation at position 485 from alanine to glutamic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue4A5, has an amino acid sequence which includes a mutation at position 408 from leucine to serine, a mutation at position 409 from tyrosine to alanine, a mutation at position 485 from alanine to histidine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue4A6, has an amino acid sequence which includes a mutation at position 408 from leucine to serine, a mutation at position 409 from tyrosine to alanine, a mutation at position 485 from alanine to histidine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue4B5, has an amino acid sequence which includes a mutation at position 408 from leucine to serine, a mutation at position 409 from tyrosine to alanine, a mutation at position 485 from alanine to asparagine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1;

Ue4B6, has an amino acid sequence which includes a mutation at position 408 from leucine to serine, a mutation at position 409 from tyrosine to alanine, a mutation at position 485 from alanine to asparagine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1;

Ue4C5, has an amino acid sequence which includes a mutation at position 408 from leucine to serine, a mutation at position 409 from tyrosine to alanine, a mutation at position 485 from alanine to lysine, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1; and

Ue4C6, has an amino acid sequence which includes a mutation at position 408 from leucine to serine, a mutation at position 409 from tyrosine to alanine, a mutation at position 485 from alanine to lysine, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1.

### 1.2.1 Construction of recombinant expression vector of KOD DNA polymerase variant

The recombinant expression vectors of different KOD DNA polymerase variants were constructed by recombing the encoding genes of the fusion proteins of different KOD DNA polymerase variants with a His-tag into the vector pD441-pelB (DNA2.0, pD441-pelB) respectively, thus obtaining individual recombinant expression vectors of different KOD DNA polymerase variants with the His-tag. The expression of such the encoding gene was directed by a signal peptide of the vector pD441-pelB.

Fusion proteins of KOD DNA polymerase variants each are of an amino acid sequence which is formed by connecting 6 His-tags at the C-terminus of the KOD DNA polymerase variant shown in a sequence A, where the sequence A is obtained by mutating some amino acid sites with specific amino acid mutations in the amino acid sequence shown in SEQ ID NO: 1.

Encoding genes of the fusion proteins of the KOD DNA polymerase variants with His-tags are respectively of a nucleotide sequence which is formed by connecting codons of 6 His-tags to the 3' end of the nucleotide sequence of the KOD DNA polymerase variant shown in a sequence B, where the sequence B is obtained by mutating some nucleotide sites with specific base mutations in the nucleotide sequence shown in SEQ ID NO: 2.For different enzymes, a tag can be added to the N-terminus or C-terminus of the amino acid sequence of enzyme for purification, and the presence or absence of the tag does not affect the property of the enzyme as known in the art, in which the tag is generally selected from those shown in Table 1.

**Table 1 Tag and its sequence for enzyme**

| Tag(s) | number | sequence |
|---|---|---|
| Poly-Arg | 5-6, general 5 | RRRRR |
| Poly-His | 2-10, general 6 | HHHHHH |
| FLAG | 8 | DYKDDDDK |
| Strep-tag II | 8 | WSHPQFEK |
| c-myc | 10 | EQKLISEEDL |

The fusion proteins of the KOD DNA polymerase variants of the present disclosure each connect with 6 His-tags at the C-terminus, and alternatively such the KOD DNA polymerase variants also may not connect with the tag at the C-terminus as known to the skilled in the art.

### 1.2.2 Construction of recombinant bacteria

Similar to the method described in 1.1.2, the recombinant expression vectors of the KOD DNA polymerase variants constructed above were respectively transfected into *E. coli* BL21 competent cells, thus obtaining positive colonies capable of expressing the fusion proteins of different KOD DNA polymerase variants respectively.

### 1.2.3 Expression and purification of fusion protein of KOD DNA polymerase variant

Similar to the method described in 1.1.3, the positive colonies expressing the fusion proteins of the KOD DNA polymerase variants were expressed and purified, thus obtaining purified fusion proteins of KOD DNA polymerase variants.

The purified fusion proteins of KOD DNA polymerase variants were detected for purity as the method in 1.1.3, and the result shows that the purity of the purified fusion proteins are all 95% or above.

### 1.3 Preparation of fusion protein of C-terminal shortened KOD DNA polymerase variant

The C-terminal shortened KOD DNA polymerase variant is the KOD DNA polymerase variant prepared in 1.2 above of which 29 amino acids at a C-terminal region was deleted. Specifically, 29 amino acids at the C-terminal region of each of variants (including Ue1A, Ue1B, Ue1C, Ue1D, Ue1E, Ue2A, Ue2B, Ue2C, Ue2D, Ue2E, Ue3A, Ue3B, Ue3C, Ue3D, Ue3E, Ue4A, Ue4B and Ue4C) were deleted, and thus obtaining the corresponding C-terminal shortened KOD DNA polymerase variants, which are named as △Ue1A, △Ue1B, △Ue1C, △Ue1D, △Ue1E, △Ue2A, △Ue2B, △Ue2C, △Ue2D, △Ue2E, △Ue3A, △Ue3B, △Ue3C, △Ue3D, △Ue3E, △Ue4A, △Ue4B and △Ue4C.

The fusion proteins of the C-terminal shortened KOD DNA polymerase variants are the C-terminal shortened KOD DNA polymerase variants described above which each connect with 6 His-tags at the C-terminus respectively.

The fusion proteins of the C-terminal shortened KOD DNA polymerase variants were prepared similar to the method in 1.2.

### Example 2. Characterization of fusion protein of KOD DNA polymerase variant

The fusion proteins of KOD DNA polymerase variants in this example are all those prepared according to method 1.2 above, which respectively contain 6 His-tags at the C-terminus.

### 2.1 polymerization activity assay of fusion protein of KOD DNA polymerase variant

The fusion proteins of KOD DNA polymerase variants were detected for polymerization activity according to the procedure described in Nishioka, M., et al. (2001. J. Biotechnol. 88), where one enzyme unit was defined as the amount of enzyme required for converting 10nmol dNTP to an acid insoluble material in 30 minutes at 75°C and a 50µL reaction system.

The reaction system for the wild-type KOD DNA polymerase includes 20 mM Tris-HCl (pH 7.5), 8 mM MgCl₂, 50 µg/mL BSA, 0.15 mM each dNTPs (dATP, dTTP, dCTP and dGTP), [methyl-3H]-TTP (0.13 mCi/nmol), 150 mg/mL activated calf thymus, 7.5 mM DTT, and 1 µL wild-type KOD DNA polymerase (1 mg/mL). The reaction system is of a total reaction volume of 50 µL.

The reaction system above was incubated at 75° for 30 minutes, and the polymerization activity of the wild-type KOD DNA polymerase was calculated to be 2 U/µL according to the definition above.

The fusion protein of wild-type KOD DNA polymerase and fusion proteins of KOD DNA polymerase variants were assayed according to the method described above.

KOD DNA polymerase variant Ue1E was detected in this example,
where Ue1E has an amino acid sequence which includes a mutation at position 408 from leucine to isoleucine, a mutation at position 409 from tyrosine to alanine, and a mutation at position 485 from alanine to glutamic acid compared to the amino acid sequence depicted in SEQ ID NO: 1; and the encoding gene of Ue1E has a nucleotide sequence which includes a mutation at position 1222 from cytosine (C) to adenine (A), a mutation at position 1224 from guanine (G) to thymine (T), a mutation at position 1225 from thymine (T) to guanine (G), a mutation at position 1226 from adenine (A) to cytosine (C), a mutation at position 1227 from thymine (T) to adenine (A) and a mutation at position 1454 from cytosine (C) to adenine (A), compared to the nucleotide sequence depicted in SEQ ID NO: 2.

The fusion proteins of the KOD DNA polymerase variants are those which each connect with 6 His-tags at the C-terminus respectively compared to their corresponding KOD DNA polymerase variants.

The results of polymerization activity of the KOD DNA polymerase variant are shown in Table2. It can be seen that the variant has DNA polymerization activity.

**Table 2: polymerization activity of KOD DNA polymerase variant**

| Names | Polymerization activity | Mutation position | Amino acide after mutation |
|---|---|---|---|
| Wild-type KOD DNA polymerase | 2 U/µL | None | None |
| Variant Ue1E | 1 U/µL | 408, 409, 485 | Isoleucine at position 408, Alanine at position 409, glutamic acid at position 485 |

The results of polymerization activity of other variants are similar to the variant Ue1E.

### 2.2 Single base incorporation kinetics of fusion protein of KOD DNA polymerase variant

In this example, the relative reaction rate of the fusion protein of the KOD DNA polymerase variant was measured by using a Cy3 fluorescent dye-labeled dATP (i.e. dATP-Cy3) and a DNA template labeled with Cy5 fluorescent dye (i.e. DNA template-Cy5), with a microplate reader detecting a fluorescence value, by which the Michaelis-Menten kinetics curve is depicted. The special experimental method is as below.

Specifically, a DNA template depicted in SEQ ID NO:7 synthesized by Genscript company was mixed with a pair of primer labeled with 5'Cy5 fluorescent dye (S1A depicted in SEQ ID NO:5, and S2A depicted in SEQ ID NO:6) in an equimolar of 1:1:1, followed by annealing at 65°C for 1 minute, at 40°C for 1 minute and at 4°C for 10 minutes, with the DNA product annealed (i.e. the DNA template-Cy5 labeled with Cy5 fluorescent dye) stored at -20°C in the dark.

The enzymatic reaction was performed by incubating a reaction system in a 384-well plate (Corning black, clear bottom 384-well plate), with the BioTek microplate reader detecting the result of enzyme activity. The reaction system is of a total reaction volume of 50µL.

The reaction system for KOD DNA polymerase variant includes 2U fusion protein of KOD DNA polymerase variant, dNTPs (1µM dATP-Cy3, 10µM dTTP, 10µM dCTP and 10 µM dGTP), the DNA template-Cy5 and an enzyme reaction buffer in a pH 8.8. The Michaelis-Menten kinetics curve of each variant was created by using the DNA template -Cy5 in 8 concentrations of 2 nmol, 4 nmol, 5 nmol, 8 nmol, 10 nmol, 20 nmol, 40 nmol and 80nmol. The temperature of the enzymatic reaction is 25°C.

The enzyme activity of the fusion protein of KOD DNA polymerase variant was measured according to the following kinetic detection mode, with data recorded once per minute.

| | |
|---|---|
| Extension | 530nm |
| Emission | 676nm |
| Reaction Temperature | 40°C |
| Reaction time | 30min |

After the enzymatic reaction was completed, data in a table form or the enzyme activity kinetics curve can be directly obtained, based on which the reaction rate of relative fluorescence can be calculated approximatively.

Since the reaction rate of relative fluorescence depends on the concentration of the DNA template-Cy5, a Km value of the fusion protein of KOD DNA polymerase variant can be determined approximatively according to the enzymatic activity detected under different concentrations of the DNA template-Cy5, where the Km value refers to the concentration of DNA template-Cy5 corresponding to half of the maximum reaction rate.

The fusion protein of the wild-type KOD DNA polymerase (KOD-WT) was used as a control.

The KOD DNA polymerase variants Ue1E, Ue1E5 and Ue1E6 were used, which were prepared as the method in Example 1.

The KOD DNA polymerase variant Ue1E has an amino acid sequence as depicted in SEQ ID NO: 8 which includes a mutation at position 408 from leucine to isoleucine, a mutation at position 409 from tyrosine to alanine, and a mutation at position 485 from alanine to glutamic acid compared to the amino acid sequence depicted in SEQ ID NO: 1; and the encoding gene of Ue1E has a nucleotide sequence as depicted in SEQ ID NO: 9 which includes a mutation at position 1222 from cytosine (C) to adenine (A), a mutation at position 1224 from guanine (G) to thymine (T), a mutation at position 1225 from thymine (T) to guanine (G), a mutation at position 1226 from adenine (A) to cytosine (C), a mutation at position 1227 from thymine (T) to adenine (A) and a mutation at position 1454 from cytosine (C) to adenine (A), compared to the nucleotide sequence depicted in SEQ ID NO: 2.

The KOD DNA polymerase variant Ue1E5 has an amino acid sequence as depicted in SEQ ID NO: 10 which includes a mutation at position 408 from leucine to isoleucine, a mutation at position 409 from tyrosine to alanine, a mutation at position 485 from alanine to glutamic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine compared to the amino acid sequence depicted in SEQ ID NO: 1; and the encoding gene of Ue1ES has a nucleotide sequence as depicted in SEQ ID NO: 11 which includes a mutation at position 1222 from cytosine (C) to adenine (A), a mutation at position 1224 from guanine (G) to thymine (T), a mutation at position 1225 from thymine (T) to guanine (G), a mutation at position 1226 from adenine (A) to cytosine (C), a mutation at position 1227 from thymine (T) to adenine (A), a mutation at position 1454 from cytosine (C) to adenine (A), a mutation at position 158 from adenine (A) to thymine (T), a mutation at position 159 from adenine (A) to guanine (G), a mutation at position 596 from adenine (A) to thymine (T), a mutation at position 597 with guanine (G), a mutation at position 727 from cytosine (C) to adenine (A), a mutation at position 728 from guanine (G) to thymine (T), a mutation at position 729 from cytosine (C) to guanine (G), a mutation at position 1577 from adenine (A) to thymine (T), a mutation at position 1578 from adenine (A) to guanine (G), a mutation at position 2021 from adenine (A) to thymine (T), a mutation at position 2125 from cytosine (C) to guanine (G), a mutation at position 2126 from guanine (G) to thymine (T), and a mutation at position 2127 from thymine (T) to guanine (G), compared to the nucleotide sequence depicted in SEQ ID NO: 2.

The KOD DNA polymerase variant Ue1E6, has an amino acid sequence as depicted in SEQ ID NO: 12 which includes a mutation at position 408 from leucine to isoleucine, a mutation at position 409 from tyrosine to alanine, a mutation at position 485 from alanine to glutamic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine compared to the amino acid sequence depicted in SEQ ID NO: 1; and the encoding gene of Ue1E6 has a nucleotide sequence as depicted in SEQ ID NO: 13 which includes a mutation at position 1222 from cytosine (C) to adenine (A), a mutation at position 1224 from guanine (G) to thymine (T), a mutation at position 1225 from thymine (T) to guanine (G), a mutation at position 1226 from adenine (A) to cytosine (C), a mutation at position 1227 from thymine (T) to adenine (A), a mutation at position 1454 from cytosine (C) to adenine (A), a mutation at position 175 from cytosine (C) to thymine (T), a mutation at position 176 from adenine (A) to thymine (T), a mutation at position 1673 from adenine (A) to thymine (T), a mutation at position 1674 from adenine (A) to guanine (G), a mutation at position 1837 from cytosine (C) to adenine (A), a mutation at position 1838 from guanine (G) to thymine (T), a mutation at position 1839 from thymine (T) to guanine (G), a mutation at position 1921 from cytosine (C) to adenine (A), a mutation at position 1922 from guanine (G) to thymine (T), a mutation at position 1923 from thymine (T) to guanine (G), a mutation at position 2012 from adenine (A) to thymine (T), a mutation at position 2013 from adenine (A) to guanine (G), a mutation at position 2018 from adenine (A) to thymine (T), and a mutation at position 2075 from adenine (A) to thymine (T), compared to the nucleotide sequence depicted in SEQ ID NO: 2.

The fusion proteins of the KOD DNA polymerase variants are those which each connect with 6 His-tags at the C-terminus respectively compared to their corresponding KOD DNA polymerase variants.

The Michaelis-Menten kinetics curves are shown in Figure 2. It can be seen, the KOD DNA polymerase variants Ue1E, Ue1E5 and Ue1E6 all exhibit better polymerization activity compared to the wild-type KOD polymerase (KOD-WT) when a single-stranded DNA was used as a template.

In addition, the results in this example indicate that a specific KOD DNA polymerase variant, whose Km value is approximate to the affinity of DNA polymerase and DNA template, remains its polymerization activity (i.e. catalytic reaction efficiency), thus being capable of realizing an ideal DNA binding affinity without affecting the specific catalytic properties. Therefore, the KOD DNA polymerase variants which have increased Km values can rapidly dissociate with DNA molecule, thus accelerating the polymerization reaction to some extent, and benefiting the elution of such the KOD DNA polymerase variant from a chip.

### 2.3 Activity assay of fusion protein of KOD DNA polymerase variant Ue1E5 on a chip

The KOD DNA polymerase variant Ue1E5 described above was selected for detecting its activity on a chip, where the variant Ue1E5 has a mutation with isoleucine at position 408, a mutation with alanine at position 409, a mutation with glutamic acid at position 485, a mutation with methionine at position 53, a mutation with methionine at position 199, a mutation with methionine at position 243, a mutation with methionine at position 526, a mutation with alanine at position 674, and a mutation with methionine at position 709.

After the DNA template depicted in SEQ ID NO:7 synthesized by Genscript company was immobilized on a biochip gencbio amino substrate (Gencbio, GCB01003), the reaction between the fusion protein of KOD DNA polymerase variant Ue1E5 and the DNA template was conducted in a non-aqueous solution according to the following procedure.

After the amino-modified chip was washed with 0.2M phosphate buffer (0.2M NaH₂PO₄ and 0.2M Na₂HPO₄, PH7.0), 30ng/µL DNA templatein an appropriate amount was spread out over the whole chip and kept at room temperature for 15 minutes for immobilization, such that the DNA template with negative charges can be sufficiently immobilized on the surface of the chip with positive charges. After that, the chip was washed again with the enzyme reaction buffer in a pH 8.8 (including 20 mM Tris-HCl, 10 mM (NH₄)₂SO₄, 10 mM KCl and 2 mM MgSO₄) at 25°C to remove the excess DNA template. Subsequently, a reaction system including 2U fusion protein of KOD DNA polymerase variant Ue1E5, 1µM dATP-Cy3, 10µM dTTP, 10µM dCTP and 10 µM dGTP was added to spread out over the whole chip and incubated at 65°C for 15 minutes, followed by washing the chip with the phosphate buffer (0.2M, PH7.0) twice to remove excess dNTPs which are unreacted. After completion, the chip was observed and photographed by a fluorescence microscope according to the detection conditions of the Cy3 fluorescent dye (530 nm of excitation wavelength and 568 nm of emission wavelength). The wild-type KOD polymerase as a control was assayed similarly.

If more and denser bright spots are present in the photo obtained, the reaction between the fusion protein of KOD DNA polymerase variant and the DNA template immobilized on the chip is more sufficient, and the KOD DNA polymerase variant is of a stronger polymerization activity.

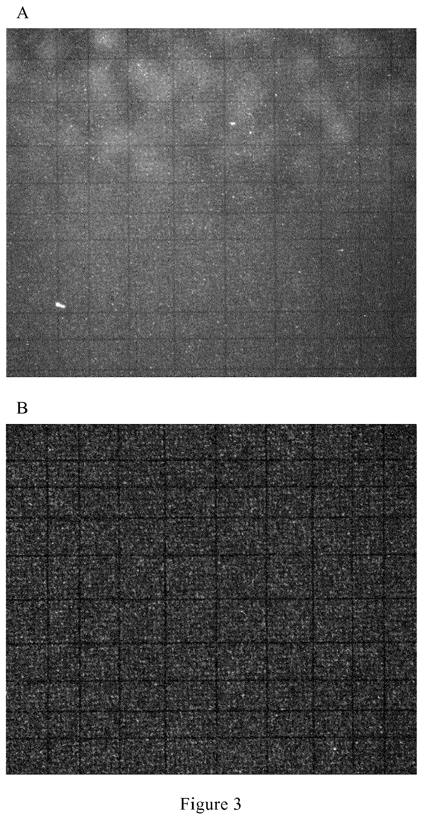

The results are shown in Figure 3, where Figure 3A indicates the result of the wild-type KOD polymerase and Figure 3B indicates the result of KOD DNA polymerase variant Ue1E5. It can be seen, few bright spots are present on the chip in Figure 3A, indicating the wild-type KOD polymerase which is unmodified or mutated exhibits a poor even no activity when reacting with the DNA template on the surface of the chip, while more and denser bright spots are present on the chip in Figure 3B, which indicates the KOD DNA polymerase variant Ue1E5 is more active than the wild-type KOD polymerase when reacting with the DNA template on the surface of the chip.

The KOD DNA polymerase variants Ue1E and Ue1E6 were also tested for their activity on a chip as the method similar to Ue1E5, with results similar to Ue1E5, which indicates a good polymerization activity for the Ue1E and Ue1E6.

The fusion proteins of the KOD DNA polymerase variants are those which each connect with 6 His-tags at the C-terminus respectively compared to their corresponding KOD DNA polymerase variants.

### 2.4 Elution assay of KOD DNA polymerase variant Ue1E5 on a chip

For an enzymatic reaction in a chip, the reaction residue in a previous step will interfere with a subsequent step, which can directly affect the photographing effect of the whole chip, thus it is ideal to remove the reaction residue left in the previous step during the enzymatic reaction.

In this example, KOD DNA polymerase variant Ue1E5 as an example was assayed for elution effect on a chip, while the commercial enzyme Therminator (Therminator^{™} DNA Polymerase, NEB, M0261S) was used as a control. The specific experimental methods are as follows.

The predetermined treatment of the chip and enzymatic reaction on the chip were conducted as the method described in 2.3.

After the photographing, the chip was washed 3 or 4 times with the enzyme reaction buffer in pH 8.8 including 20 mM Tris-HCl, 10 mM (NH₄)₂SO₄, 10 mM KCl and 2 mM MgSO₄ at 25°C, followed by incubating in a reaction system which contains dNTPs and the enzyme reaction buffer but without the DNA polymerase (i.e. the KOD DNA polymerase variant or the Therminator) at 65°C for 15 minutes. After completion, the chip was washed with the enzyme reaction buffer twice, and further observed and photographed by the fluorescence microscope according to the detection conditions of the Cy3 fluorescent dye (530 nm of excitation wavelength and 568 nm of emission wavelength).

In this experiment, more bright spots present in a photo obtained means that the enzymatic reaction continues, which indicates the KOD DNA polymerase variant used still remains on the chip, suggesting such the variant is not easy to be eluted out.

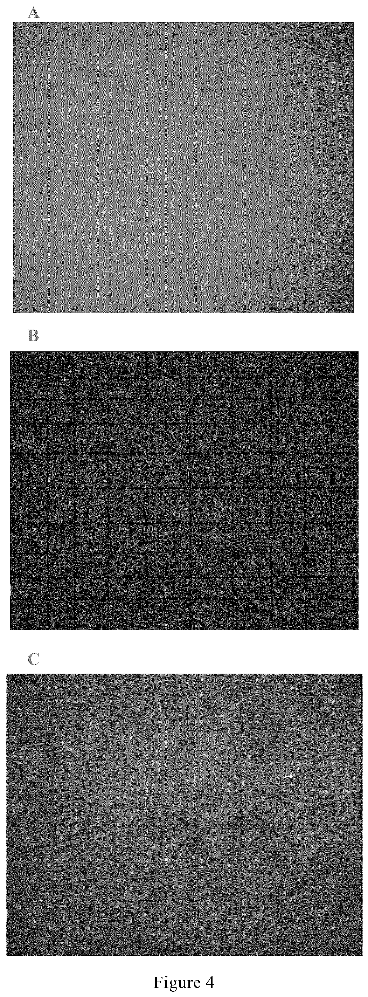

The experimental results are shown in Figure 4, where Figure 4A shows a blank control, i.e. a blank chip, Figure 4B indicates the result of the Therminator, and Figure 4C indicates the result of KOD DNA polymerase variant Ue1E5. It can be seen, no bright spots are present on the blank chip of Figure 4A due to no enzymatic reaction, more bright spots are present in Figure 4B compared to the blank control, and few bright spots are present in Figure 4C which are more than those in the blank control and less than those shown in Figure 4B, which indicates the KOD DNA polymerase variant Ue1E5 achieves a better elution effect than the Therminator, suggesting such the variant is easy to be eluted out, and is poor at adsorbing on the surface of the chip, thus without interference to subsequent steps.

The wild-type KOD DNA polymerase cannot be used for elution assay due to its poor polymerization activity, thus the commercial enzyme Therminator was used as a control.

The fusion protein of KOD DNA polymerase variant Ue1E6 was assayed according to the similar method, with a result not significantly different with Ue1E5, showing Ue1E6 is easier to be eluted out than Therminator.

Other KOD DNA polymerase variants were detected according to the similar method, and the results show all of them are easy to be eluted out and are poor at adsorbing on the surface of the chip.

### Example 3: Activity assay of fusion protein of C-terminal shortened KOD DNA polymerase variant on a chip

The fusion proteins of the KOD DNA polymerase variants are those which each connect with 6 His-tags at the C-terminus respectively compared to their corresponding KOD DNA polymerase variants.

The fusion proteins of C-terminal shortened KOD DNA polymerase variants prepared according to the method in 1.3 (including △Ue1A, △Ue1B, △Ue1C, △Ue1D, △Ue1E, △Ue2A, △Ue2B, △Ue2C, △Ue2D, △Ue2E, △Ue3A, △Ue3B, △Ue3C, △Ue3D, △Ue3E, △Ue4A, △Ue4B and △Ue4C) were respectively assayed for their polymerization activity as the method in 2.1 and their activity on a chip as the method in 2.3.

The fusion proteins of KOD DNA polymerase variants including Ue1A, UelB, Ue1C, UelD, UelE, Ue2A, Ue2B, Ue2C, Ue2D, Ue2E, Ue3A, Ue3B, Ue3C, Ue3D, Ue3E, Ue4A, Ue4B and Ue4Cwere respectively used as controls.

The results of the fusion proteins of C-terminal shortened KOD DNA polymerase variants (including △Ue1A, △Ue1B, △Ue1C, △Ue1D, △Ue1E, △Ue2A, △Ue2B, △Ue2C, △Ue2D, △Ue2E, △Ue3A, △Ue3B, △Ue3C, △Ue3D, △Ue3E, △Ue4A, △Ue4B and △Ue4C) are respectively not significantly different with the results of corresponding fusion proteins of KOD DNA polymerase variants (including Ue1A, Ue1B, Ue1C, Ue1D, Ue1E, Ue2A, Ue2B, Ue2C, Ue2D, Ue2E, Ue3A, Ue3B, Ue3C, Ue3D, Ue3E, Ue4A, Ue4B and Ue4C).

The results demonstrated that deletion of 29 amino acids at the C-terminal region of KOD DNA polymerase variant does not affect the activity of the variant.

The fusion proteins of C-terminal shortened KOD DNA polymerase variants in this example were respectively assayed according to the method in example 2.4, with the results showed that such the C-terminal shortened variants are not easy to be eluted out and are poor at adsorbing on the surface of the chip.

### Industrial Application

The examples of the present disclosure demonstrated that the DNA polymerase variants prepared in the present disclosure are capable of catalytically reacting with artificially modified nucleotides or their analogs as a substrate, with excellent polymerization activity, poor adsorption on the surface of a chip, and easily eluted from the surface of the chip, thereby allowing a single nucleotide or its analogs to be added in each reaction cycle, without interfering with the subsequent sequencing cycle due to its excellent elution property.

## Claims

1. A protein that has DNA polymerase activity and which is **characterised by** having improved catalytic and physicochemical properties in comparison to a wild-type KOD DNA polymerase of SEQ ID NO: 1, and
wherein the protein has an amino acid sequence which includes a mutation at position 408 from leucine to isoleucine, a mutation at position 409 from tyrosine to alanine, a mutation at position 485 from alanine to glutamic acid, a mutation at position 53 from lysine to methionine, a mutation at position 199 from lysine to methionine, a mutation at position 243 from arginine to methionine, a mutation at position 526 from lysine to methionine, a mutation at position 674 from lysine to alanine, and a mutation at position 709 from arginine to methionine as compared to an amino acid sequence of the wild-type KOD DNA polymerase of SEQ ID NO: 1; or has an amino acid sequence which includes a mutation at position 408 from leucine to isoleucine, a mutation at position 409 from tyrosine to alanine, a mutation at position 485 from alanine to glutamic acid, a mutation at position 59 from histidine to phenylalanine, a mutation at position 558 from lysine to methionine, a mutation at position 613 from arginine to methionine, a mutation at position 641 from arginine to methionine, a mutation at position 671 from lysine to methionine, a mutation at position 673 from tyrosine to phenylalanine, and a mutation at position 692 from lysine to methionine as compared to an amino acid sequence of the wild-type KOD DNA polymerase of SEQ ID NO: 1.

2. The protein according to claim 1, wherein the protein is modified by deleting amino acids 1 to 29 from a C-terminus of the protein and keeping the remaining amino acids unchanged.

3. The protein according to claim 1 or 2, wherein the protein is modified by adding a tag to an N-terminus or C-terminus of the amino acid sequence of the protein.

4. The protein according to claim 3, wherein the tag is selected from Poly-Arg, Poly-His, FLAG, Strep-tag II and c-myc as shown in Table 1:
| Tag(s) | number | sequence |
|---|---|---|
| Poly-Arg | 5-6, general 5 | RRRRR |
| Poly-His | 2-10, general 6 | HHHHHH |
| FLAG | 8 | DYKDDDDK |
| Strep-tag II | 8 | WSHPQFEK |
| c-myc | 10 | EQKLISEEDL |

5. A DNA molecule encoding the protein according to any one of claims 1 to 4, or an expression cassette, a recombinant expression vector, a recombinant bacteria or a transgenic cell line containing the DNA molecule.

6. Use of the protein according to any one of claims 1 to 4, or the DNA molecule, the expression cassette, the recombinant expression vector, the recombinant bacteria or the transgenic cell line according to claim 5 for preparing a DNA polymerase.

7. Use of the protein according to any one of claims 1 to 4, or the DNA molecule, the expression cassette, the recombinant expression vector, the recombinant bacteria or the transgenic cell line according to claim 5 in sequencing.

8. Use of the protein according to any one of claims 1 to 4, or the DNA molecule, the expression cassette, the recombinant expression vector, the recombinant bacteria or the transgenic cell line according to claim 5 in preparing a kit for sequencing.

## Patentansprüche

1. Ein Protein, das DNA-Polymerase-Aktivität aufweist und **dadurch gekennzeichnet ist, dass** es im Vergleich zu einer Wildtyp-KOD-DNA-Polymerase der SEQ ID NO: 1 verbesserte katalytische und physikalisch-chemische Eigenschaften aufweist, und
wobei das Protein eine Aminosäuresequenz aufweist, die eine Mutation an Position 408 von Leucin zu Isoleucin, eine Mutation an Position 409 von Tyrosin zu Alanin, eine Mutation an Position 485 von Alanin zu Glutaminsäure, eine Mutation an Position 53 von Lysin zu Methionin, eine Mutation an Position 199 von Lysin zu Methionin, eine Mutation an Position 243 von Arginin zu Methionin, eine Mutation an Position 526 von Lysin zu Methionin, eine Mutation an Position 674 von Lysin zu Alanin und eine Mutation an Position 709 von Arginin zu Methionin im Vergleich zu einer Aminosäuresequenz der Wildtyp-KOD-DNA-Polymerase der SEQ ID NO: 1 einschließt; oder eine Aminosäuresequenz aufweist, die eine Mutation an Position 408 von Leucin zu Isoleucin, eine Mutation an Position 409 von Tyrosin zu Alanin, eine Mutation an Position 485 von Alanin zu Glutaminsäure, eine Mutation an Position 59 von Histidin zu Phenylalanin, eine Mutation an Position 558 von Lysin zu Methionin, eine Mutation an Position 613 von Arginin zu Methionin, eine Mutation an Position 641 von Arginin zu Methionin, eine Mutation an Position 671 von Lysin zu Methionin, eine Mutation an Position 673 von Tyrosin zu Phenylalanin und eine Mutation an Position 692 von Lysin zu Methionin im Vergleich zu einer Aminosäuresequenz der Wildtyp-KOD-DNA-Polymerase der SEQ ID NO: 1 einschließt.

2. Protein nach Anspruch 1, wobei das Protein modifiziert ist, indem die Aminosäuren 1 bis 29 vom C-Terminus des Proteins entfernt und die verbleibenden Aminosäuren unverändert belassen werden.

3. Protein nach Anspruch 1 oder 2, wobei das Protein modifiziert ist, indem ein Tag an den N-Terminus oder C-Terminus der Aminosäuresequenz des Proteins angefügt wird.

4. Protein nach Anspruch 3, wobei das Tag aus Poly-Arg, Poly-His, FLAG, Strep-Tag II und c-myc ausgewählt ist, wie in Tabelle 1 gezeigt:
| Tag(s) | Nummer | Sequenz |
|---|---|---|
| Poly-Arg | 5-6, general 5 | RRRRR |
| Poly-His | 2-10, general 6 | HHHHHH |
| FLAG | 8 | DYKDDDDK |
| Strep-tag II | 8 | WSHPQFEK |
| c-myc | 10 | EQKLISEEDL |

5. Ein DNA-Molekül, das für das Protein gemäß einem der Ansprüche 1 bis 4 kodiert, oder eine Expressionskassette, ein rekombinanter Expressionsvektor, ein rekombinantes Bakterium oder eine transgene Zelllinie, die das DNA-Molekül enthält.

6. Verwendung des Proteins gemäß einem der Ansprüche 1 bis 4 oder des DNA-Moleküls, der Expressionskassette, des rekombinanten Expressionsvektors, des rekombinanten Bakteriums oder der transgenen Zelllinie gemäß Anspruch 5 zur Herstellung einer DNA-Polymerase.

7. Verwendung des Proteins gemäß einem der Ansprüche 1 bis 4 oder des DNA-Moleküls, der Expressionskassette, des rekombinanten Expressionsvektors, des rekombinanten Bakteriums oder der transgenen Zelllinie gemäß Anspruch 5 bei der Sequenzierung.

8. Verwendung des Proteins gemäß einem der Ansprüche 1 bis 4 oder des DNA-Moleküls, der Expressionskassette, des rekombinanten Expressionsvektors, der rekombinanten Bakterien oder der transgenen Zelllinie gemäß Anspruch 5 bei der Herstellung eines Kits zur Sequenzierung.

## Revendications

1. Protéine qui a une activité ADN polymérase et qui est **caractérisée en ce qu'**elle présente des propriétés catalytiques et physicochimiques améliorées par rapport à une ADN polymérase KOD de type sauvage de SEQ ID NO: 1, et
dans laquelle la protéine a une séquence d'acides aminés qui comprend une mutation en position 408 de leucine en isoleucine, une mutation en position 409 de tyrosine en alanine, une mutation en position 485 d'alanine en acide glutamique, une mutation en position 53 de lysine en méthionine, une mutation en position 199 de lysine en méthionine, une mutation en position 243 d'arginine en méthionine, une mutation en position 526 de lysine en méthionine, une mutation en position 674 de lysine en alanine et une mutation en position 709 d'arginine en méthionine par rapport à une séquence d'acides aminés de l'ADN polymérase KOD de type sauvage de SEQ ID NO: 1 ; ou a une séquence d'acides aminés qui comprend une mutation en position 408 de leucine en isoleucine, une mutation en position 409 de tyrosine en alanine, une mutation en position 485 d'alanine en acide glutamique, une mutation en position 59 d'histidine en phénylalanine, une mutation en position 558 de lysine en méthionine, une mutation en position 613 d'arginine en méthionine, une mutation en position 641 d'arginine en méthionine, une mutation en position 671 de lysine en méthionine, une mutation en position 673 de tyrosine en phénylalanine et une mutation en position 692 de lysine en méthionine par rapport à une séquence d'acides aminés de l'ADN polymérase KOD de type sauvage de SEQ ID NO: 1.

2. Protéine selon la revendication 1, dans laquelle la protéine est modifiée par suppression des acides aminés 1 à 29 à partir d'une extrémité C-terminale de la protéine, les acides aminés restants étant conservés inchangés.

3. Protéine selon la revendication 1 ou 2, dans laquelle la protéine est modifiée par ajout d'une étiquette à une extrémité N-terminale ou C-terminale de la séquence d'acides aminés de la protéine.

4. Protéine selon la revendication 3, dans laquelle l'étiquette est choisie parmi Poly-Arg, Poly-His, FLAG, Strep-tag II et c-myc comme indiqué dans le tableau 1 :
| Étiquette(s) | nombre | séquence |
|---|---|---|
| Poly-Arg | 5-6, en général 5 | RRRRR |
| Poly-His | 2-10, en général 6 | HHHHHH |
| FLAG | 8 | DYKDDDDK |
| Strep-tag II | 8 | WSHPQFEK |
| c-myc | 10 | EQKLISEEDL |

5. Molécule d'ADN codant pour la protéine selon l'une quelconque des revendications 1 à 4, ou cassette d'expression, vecteur d'expression recombinant, bactérie recombinante ou lignée cellulaire transgénique contenant la molécule d'ADN.

6. Utilisation de la protéine selon l'une quelconque des revendications 1 à 4, ou de la molécule d'ADN, de la cassette d'expression, du vecteur d'expression recombinant, de la bactérie recombinante ou de la lignée cellulaire transgénique selon la revendication 5 pour la préparation d'une ADN polymérase.

7. Utilisation de la protéine selon l'une quelconque des revendications 1 à 4, ou de la molécule d'ADN, de la cassette d'expression, du vecteur d'expression recombinant, de la bactérie recombinante ou de la lignée cellulaire transgénique selon la revendication 5 dans le séquençage.

8. Utilisation de la protéine selon l'une quelconque des revendications 1 à 4, ou de la molécule d'ADN, de la cassette d'expression, du vecteur d'expression recombinant, de la bactérie recombinante ou de la lignée cellulaire transgénique selon la revendication 5 dans la préparation d'un kit de séquençage.
